# EUROPEAN PATENT APPLICATION

(11) **EP 4 640 242 A1**
(43) Date of publication of application: **29.10.2025**
(21) Application number: 25158281.3
(22) Date of filing: 16.03.2021
(51) Int. Cl.: A61L 2/10, A47L 11/40, A61L 2/24, A61L 9/20

(54) **REMOTELY OR AUTONOMOUSLY OPERABLE DISINFECTION APPARATUS**

(30) Priority: 16.03.2020 GB 202003782; 24.11.2020 GB 202018483
(62) Divisional of application: 21716270.0
(71) Applicant: Akara Robotics Limited, Dublin 8 (IE)
(72) Inventor: BURKE, Eamonn, Dublin (IE); CULLINAN, Michael, Dublin (IE); MCGINN, Conor, Dublin (IE)
(74) Representative: Purdylucey Intellectual Property

(57) **Abstract**

A disinfection apparatus with a germicidal radiation source, a moveable support to which the germicidal light source is attached, a controller for controlling emission of germicidal light radiated omnidirectionally from the germicidal light source. The controller has a germicidal light blocker and a control system so as to selectively block light emitted from the light source in selected directions and direct it in other directions. The germicidal radiation blocker has a first barrier which is fixed in position with respect, and moves with the germicidal radiation source and has a reflecting surface which reflects germicidal radiation in a direction to and out from a second side of the germicidal radiation source, the radiation source and first barrier being rotatable relative to a second barrier which is fixed relative to the movable support wherein the first barrier and the second barrier work together to completely or partially encapsulate the light source with respect to its surrounding environment.

## Description

### Field

The present invention relates to a disinfection apparatus for disinfecting objects such as floors, surfaces, air, equipment and furniture. In particular the present invention relates to an autonomous, semi-autonomous or remotely controlled disinfection apparatus.

### Background

The prevention and control of infection is extremely important in all locations but is of particular importance in facilities where people are particularly vulnerable to the health consequences of infection, such as hospitals, clinics and care homes.

In such locations a high standard of infection control is required to control the amount and spread of micro-organisms that cause infection and disease. These microorganisms, known as pathogens or infectious agents include:
Bacteria e.g. staphylococcal aureus (found nearly everywhere in the environment i.e. soil, air);
Viruses can survive out of the body for a time;
Pathogenic Fungi such as moulds or yeast; and
Parasites which may cause infection and are spread from person to person.

Micro-organisms which cause infection may originate from one's self (endogenous), other people (exogenous) and the environment.

Micro-organisms are always present in the environment and there is a responsibility to ensure that inanimate objects such as furniture, wheelchairs, re-usable medical devices and so on in a facility are decontaminated properly to minimise the risk of infection to residents, staff and visitors. Decontamination is a general term for the destruction or removal of microbial contamination to render an item safe.

UV light is electromagnetic radiation with wavelengths shorter than visible light but longer than X-rays. UV can be separated into various ranges, with short-wavelength UV (UVC) being referred to, in this context, as ultraviolet germicidal irradiation (UVGI). UVC wavelengths between about 100 nm and 300 nm are strongly absorbed by nucleic acids. The absorbed energy can result in a disruption to the operation of their DNA by for example, creating defects including pyrimidine dimers. These dimers can prevent replication or can prevent the expression of necessary proteins, resulting in the death or inactivation of the micro-organism.

Suitable sources of UV which can operate as a disinfectant include Mercury-based lamps which emit UV light at the 253.7 nm line, Ultraviolet light-emitting diodes (UV-C LED) which emit UV light at selectable wavelengths between 255 and 280 nm, pulsed-xenon which have a peak emission near 230 nm, and Far UV systems which emit at around 222nm. The effectiveness of germicidal UV depends on the length of time a microorganism is exposed to UV, the intensity and wavelength of the UV radiation, the presence of particles that can protect the microorganisms from UV, and a microorganism's ability to withstand UV during its exposure.

Effectiveness also depends on line-of-sight exposure of the microorganisms to the UV light. Environments where design creates obstacles that block the UV light are not as effective. In such an environment, the effectiveness is then reliant on the placement of a UVGI system so that line of sight is optimum for disinfection.

It is known to mount a UVGI source on a moveable base to allow it to move around a room. For example, UVD Robots Aps have created a product described as a UV disinfection robot. The device comprises a moveable base upon which is mounted a number of UV lamps. The device also has an autonomous navigation system to allow it to move from room to room in a facility and to move around rooms it is cleaning. It also has hospital integration and reporting software. A device produced by Xenex Disinfection Services LLC uses pulsed xenon light which is mounted on a trolley. The Mediland disinfection device is mounted on wheels but is manually moved from location to location.

The following disadvantages have been identified in one or more of the above devices.
- They cannot block direct UVC radiation from a person in the same room as the device, therefore, the room must be empty when the device is in use.
- No easy way to communicate with a person in the vicinity or detect their presence.
- The device is not wireless
- The device has no navigation capability

US2008 056933, CN209529745, WO2014039076, US2017112954, US2016271803 and CN208705724 have been identified as examples of the state of the art. A problem with prior art systems is that when a person enters a room or area where the disinfection apparatus is in operation a person walking into the room or area can be exposed to harmful radiation without that person's knowledge.

It is an object of the present invention to provide an apparatus and method for using UV light to disinfect/sanitise objects and regions of the room from harmful microorganisms such as fungi, viruses and bacteria.

It is an object of the invention to overcome at least one of the above-referenced problems.

### Summary

According to the invention there is provided a disinfection apparatus, and a method for controlling the apparatus, as set out in the appended claims.

In accordance with a first aspect of the invention there is provided a disinfection apparatus which comprises:
a germicidal light source;
a controller for controlling emission of germicidal light from the germicidal light source; and
a moveable support to which the germicidal light source is attached which is moveable across a surface.

In at least one embodiment the controller further comprises a detector which detects the presence of an object in the vicinity of the apparatus wherein the controller controls the direction of germicidal light from the light source in response to the detection of one or more said object.

In at least one embodiment, the controller comprises a germicidal light blocker and a control system.

In at least one embodiment, the germicidal light blocker comprises a barrier.

In at least one embodiment, the barrier is positioned at a first side of the germicidal light source to prevent the emission of germicidal light beyond the first side.

In at least one embodiment, the barrier has a reflecting surface which reflects germicidal light in a direction to and out from a second side of the germicidal light source.

In at least one embodiment, the germicidal light blocker further comprises a enclosure which is positionable at the second side of the germicidal light source and is moveable so that when the blocking barrier is rotated to face it to the light source is partially or fully enclosed.

In at least one embodiment, the germicidal light blocker further comprises a enclosure which is fixed on the moveable base so that when the blocking barrier is rotated to face it to the light source is partially or fully enclosed.

In at least one embodiment, the enclosure may fully enclose the germicidal light source so as to prevent emission of germicidal light from the apparatus.

In at least one embodiment, the enclosure is rotatable with respect to the germicidal light source.

In at least one embodiment, the enclosure is rotatable with respect to the moveable support

In at least one embodiment, the detector detects the distance of an object from the apparatus.

In at least one embodiment, the germicidal light blocker is of rigid construction to protect the germicidal light source from physical damage.

In at least one embodiment, the controller comprises or more proximity detector which measures the position of and distance between an object and the apparatus, said distance and position measurements are used to control the direction in which germicidal light is emitted from the light source such that the germicidal light is concentrated on the object.

Advantageously, the germicidal effect of the germicidal light will be more concentrated on objects which are closer to the germicidal light source.

In another embodiment, the germicidal light source comprises a plurality of LEDs arranged on a support surface, wherein the controller comprises a switch which controls the operation of the LEDs.

In another embodiment, the blocker is positioned to ensure the germicidal light is emitted in a direction towards the object to be sterilised.

In another embodiment the germicidal light source comprises a plurality of fixed light sources positioned on a support column and facing in multiple directions.

In at least one embodiment, the controller selectively switches on the germicidal light sources facing the desired direction. This offers the advantages of mechanical simplicity and the ability to activate light sources facing multiple directions simultaneously but increases the number of light sources required on the apparatus.

In at least one embodiment, the one or more germicidal light source in closest proximity to the object to be sterilised are switched on.

In at least one embodiment, the germicidal light source is configured to selectively illuminate an area around the apparatus.

The invention concerns a disinfection apparatus which, in at least one embodiment comprises a robotic system.

In at least one embodiment the germicidal light source is UV light.

In at least one embodiment the UV light is UVC light of wavelength in the range 200 to 300nm.

In at least one embodiment, the moveable support comprises a base.

In at least one embodiment, the moveable support comprises wheels mounted for contact with a surface across which the apparatus can travel.

In at least one embodiment, the controller controls the movement of the apparatus in response to inputs from the navigation sensors.

In at least one embodiment, the apparatus comprises a robotic system.

In at least one embodiment, the robotic system is autonomous.

In at least one embodiment, the robotic system is semi-autonomous.

In at least one embodiment, the robotic system is remotely controlled.

In at least one embodiment, the robotic system is manually controlled.

In at least one embodiment, the germicidal light source comprises one or more UV lamps.

In at least one embodiment, the germicidal light source comprises one or more fluorescent lamp, LED or Xenon lamp.

In at least one embodiment, the UV lamps are mounted above the moveable support which acts as a base.

In at least one embodiment, the sensor comprises a camera.

In at least one embodiment, the sensor comprises a passive infra-red camera.

In at least one embodiment, the sensor may comprise a RGBD camera or a LIDAR (LIght Detection And Ranging) sensor

In at least one embodiment, the sensor comprises a thermal vision sensor.

In at least one embodiment, the apparatus further comprises a communication interface.

In at least one embodiment, the communication interface is a computer interface.

In at least one embodiment the computer interface is a computer screen, tablet computer or robotic head.

In at least one embodiment, the communications interface is mounted on an outer part of a germicidal light source shield.

In at least one embodiment the communication interface is fixed to the **UV** shield.

In at least one embodiment, the communications interface is connected via a movable articulated link.

In at least one embodiment, the communications interface has an audio input and/or output.

In accordance with a second aspect of the invention there is provided a method for controlling the apparatus of the first aspect in order to disinfect an object, the method comprising:
Detecting the position and distance of one or more objects with respect to the apparatus;
Determining which of said objects are closest to the apparatus;
Controlling the output of a germicidal light source such that germicidal light is directed towards said closest object.

In at least one embodiment, a barrier is positioned at a first side of the germicidal light source to prevent the emission of germicidal light beyond the first side.

In at least one embodiment, the barrier has a reflecting surface which reflects germicidal light in a direction to and out from a second side of the germicidal light source.

In at least one embodiment, the step of controlling comprises actuating a moveable enclosure positionable at the second side of the germicidal light source and moving the enclosure to partially or fully enclose the germicidal light source to control the direction in which germicidal light is emitted from the germicidal light source in the second direction.

In at least one embodiment, the enclosure may fully enclose the germicidal light source so as to prevent emission of germicidal light from the apparatus.

In at least one embodiment, the enclosure is rotatable with respect to the germicidal light source.

In at least one embodiment, the enclosure is rotatable with respect to the moveable support

In at least one embodiment, the detector detects the distance of an object from the apparatus.

In at least one embodiment, the detector determines the type of object.

In at least one embodiment, the germicidal light blocker is of rigid construction to protect the germicidal light source from physical damage.

In at least one embodiment, the controller comprises of one or more proximity detector which measures the position of and distance between an object and the apparatus, said distance and position measurements are used to control the direction in which germicidal light is emitted from the light source such that the germicidal light is concentrated on the object.

Advantageously, the germicidal effect of the germicidal light will be more concentrated on objects which are closer to the germicidal light source.

In another embodiment, the germicidal light source comprises a plurality of LEDs arranged on a support surface, wherein the controller comprises a switch which controls the operation of the LEDs.

In another embodiment, the blocker is positioned to ensure the germicidal light is emitted in a direction towards the object to be sterilised.

In another embodiment the germicidal light source comprises a plurality of fixed light sources positioned on a support column and facing in multiple directions.

In at least one embodiment, the controller selectively switches on the germicidal light sources facing the desired direction. This offers the advantages of mechanical simplicity and the ability to activate light sources facing multiple directions simultaneously.

In at least one embodiment, the one or more LED in closest proximity to the object to be sterilised are switched on.

In accordance with a third aspect of the invention there is provided a computer program with program instructions for implementing the method of the invention as herein defined.

Other aspects and preferred embodiments of the invention are defined and described in the other claims set out below.

### Brief Description of the Figures

Figures 1a to 1 c are schematic plan views of a first embodiment of an apparatus in accordance with the present invention shown in three distinct configurations;
Figure 2 is a perspective view of a second embodiment of the present invention;
Figures 3a, 3c and 3e are perspective views of a third embodiment of the present invention, figures 3b, 3d and 3f are respective plan views and figures 3g and 3h are side and front views of the same embodiment;
Figures 4a is a perspective views and 4b is a plan view of a fourth embodiment of the present invention;
Figure 5 is a front perspective view of a fifth embodiment of the present invention;
Figure 6 is a front perspective view of a sixth of the present invention;
Figure 7 is a front perspective view of a seventh embodiment of the present invention
Figure 8 is a plan view of an eighth embodiment of the present invention;
Figure 9 is a plan view of a ninth embodiment of the present invention;
Figure 10 is a plan view of a tenth embodiment of the present invention;
Figure 11 is a plan view of an eleventh embodiment of the present invention;
Figures 12 a to 12c show the operation of an apparatus in accordance with the present invention; and
Figure 13 is a flow diagram of an example of the method for controlling the apparatus in accordance with the present invention implemented on computing means.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

One object of the invention is to create a device which has a germicidal light mounted in any combination of orientations which is attached to a moveable support or base and which uses any type of UV lighting technology, for example, fluorescent lamps, LEDs or Xenon lamps. The present invention provides for the controlled emission of germicidal light from the germicidal light source such that the direction of germicidal light from the light source is controlled in response to the detection of one or more object in the path of the device. The controlled emission may prevent germicidal light from being incident on the object, if it is, for example, a person, or may concentrate the germicidal light on the object, if the object is, for example, a piece of equipment or furniture which needs to be sterilised.

The preferred implementation uses light sources such as fluorescent lamps, which radiate light omnidirectionally. In this implementation, the control of directionality of the light is achieved by positioning a blocking barrier around the light source(s) on the robot. The blocking barrier prevents the light from the light source(s) from exiting the robot except for in the selected direction. Thus, the UV light can irradiate in any given direction both independently of the orientation of the mobile base and without the requirement to move the base. It is preferred that the blocking barrier be a UVC reflector. The use of a reflector serves the dual purpose of blocking light from being emitted in the undesired direction while also concentrating the light power in the desired direction.

Another embodiment adds an enclosure system to the barrier which may partially or completely encapsulate the light source with respect to its surrounding environment. This can be realised by adding an enclosure column running parallel to the light source and blocking barrier assembly. The blocking barrier can then be orientated such that the combination of the blocking barrier and enclosure column separate the light source from the external environment.

The base may comprise wheels, navigation sensors and associated software to enable autonomous, semi-autonomous or manually controlled navigation. The device may be an autonomous, semi-autonomous or remotely controlled robot.

The device has a controller which allows the selective direction of light from the UV source in a preferred direction. The direction will be controlled autonomously, semi autonomously or manually. The controller can be used while the device is stationary, at multiple selected waypoints within its environment, or as the device moves through the area to be treated. In at least one embodiment, the device has a combination of sensors which may include cameras, passive infra-red, thermal vision sensors, etc. and associated computer software for detecting and tracking the presence of people in its vicinity.

Another embodiment adds the ability for the device to dynamically adjust the shape of the blocking barrier in order to change the angle over which light is irradiated. In another embodiment the light source and base do not rotate relative to each other, but instead the blocking barrier rotates around them in order to direct light in the selected direction. The blocking barrier can automatically orientate such that when a person enters an area the apparatus can orientate such that the barrier protects the person from harmful radiation. Moreover a communications interface mounted on the barrier becomes a focal point through which a person/operator can interact/communicate with the apparatus. The communication interface can be adapted with video capability to show a video stream of the region in front of the apparatus, giving the operator improved situational awareness.

Another implementation uses a light source which is already directional (e.g. LEDs). In this implementation the light source must be pointed towards the targeted surfaces, the blocking surface/reflector provides additional shielding.

Another embodiment incorporates fixed light sources positioned facing multiple directions on the robot. Selective emission of radiation is achieved by only illuminating the light sources facing in the desired direction. This offers the advantages of mechanical simplicity and the ability to activate light sources facing multiple directions simultaneously but increases the number of light sources required on the robot.

The light power absorbed by surfaces decreases as the light source is further away (following approximately an inverse square law). Therefore, as the device moves around a room it is advantageous to position it as close as possible to the surfaces to be irradiated. If the device radiates light in all directions it is likely that in many situations most of the light will fall on surfaces far from the robot (and therefore with less light power). With a directional light source, if all (or the majority) of the light power can be focused on surfaces close to it using a reflective blocking barrier, more light power will be absorbed by the exposed surfaces. This increases the advantage offered by moving the robot through its environment.

Avoiding irradiating light in selected directions allows the device to protect specified objects/surfaces from UVC irradiation. This may be desirable as certain materials can be degraded by prolonged exposure to UVC or for regulatory reasons. UVC is harmful to humans, affecting both skin and eyes. Ensuring humans are not exposed to UVC normally means that UVC robots cannot operate in the vicinity of people and so rooms must be evacuated prior to the device's operation.

Selective irradiation affords the ability to work alongside humans by directing UVC light only in directions in which the light will not be incident on people.

The sensors located on the device enable algorithms which can detect where people are located and ensure that the robot will not emit light towards them. As UVC light is reflected poorly by most surfaces, this approach can effectively protect people surrounding the robot from the UVC irradiation. UVC sensors on the device and/or in the environment and/or on wearables worn by people in the room may be used to verify this with the option to turn off the light sources still being available where necessary.

The preferred implementation with an enclosure column allows the light source to be protected from the outside world for transport and as the robot is moved between areas to be treated. By doing this, the light source cannot be damaged by people or objects in the robot's vicinity. This is advantageous for light sources such as fluorescent lamps which are hazardous if broken.

An enclosure column also allows complete light encapsulation so that if the light source is activated, no light is emitted to the surrounding environment. This can be useful for certain light sources which require some time to reach their nominal light output (e.g. fluorescent lamps). If it is desired that a space be disinfected as quickly as possible, the light source could be turned on in advance (i.e. before the start of the cleaning cycle), for maximum effectiveness when the disinfection cycle begins.

Additionally, should it become desirable to turn off the lights during a cleaning cycle (e.g. because people are detected in the robot's vicinity), instead of turning off the light source (which would result in a warm up period when the lamps are turned on again), the light source can be temporarily encapsulated, blocking off light to the environment. Similarly, if the robot must move from one room to be treated to another, it may be desirable to keep the lights on during this time while blocking the light output.

Shadowing (whereby light is blocked from reaching a surface to be treated by objects in the environment) is a problem for UVC disinfection. A preferred implementation allows the directionality of the light to be altered while the robot remains stationary, thus reducing this effect.

In at least one embodiment, the present invention comprises a mobile robot that uses a germicidal light source to disinfect/sanitise surfaces around it from harmful microorganisms (fungi, virus, bacteria, etc).

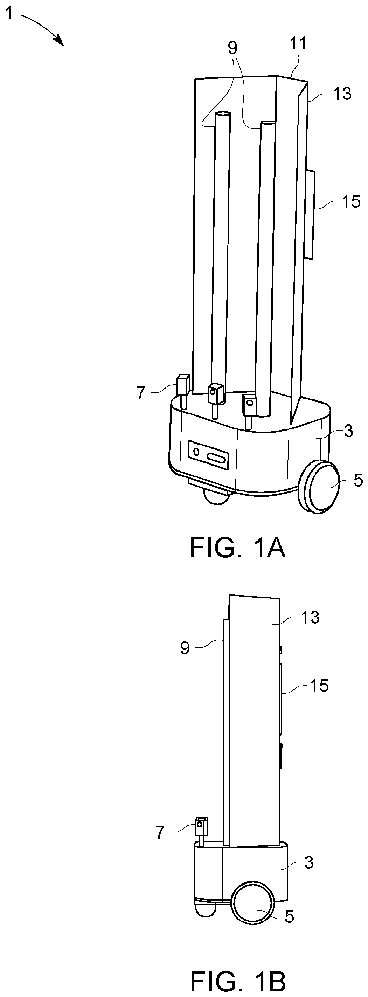

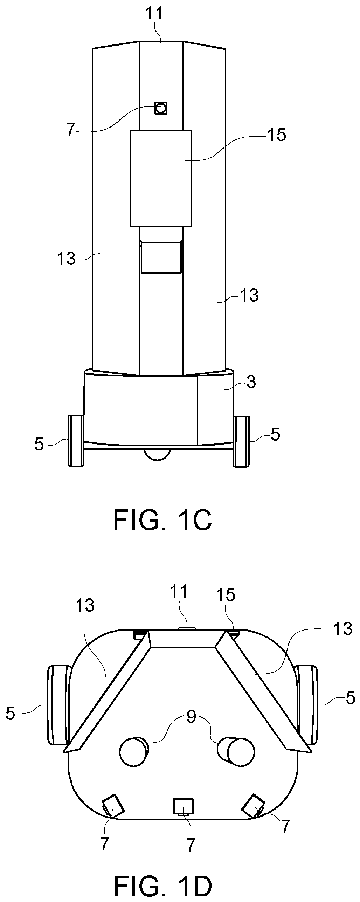

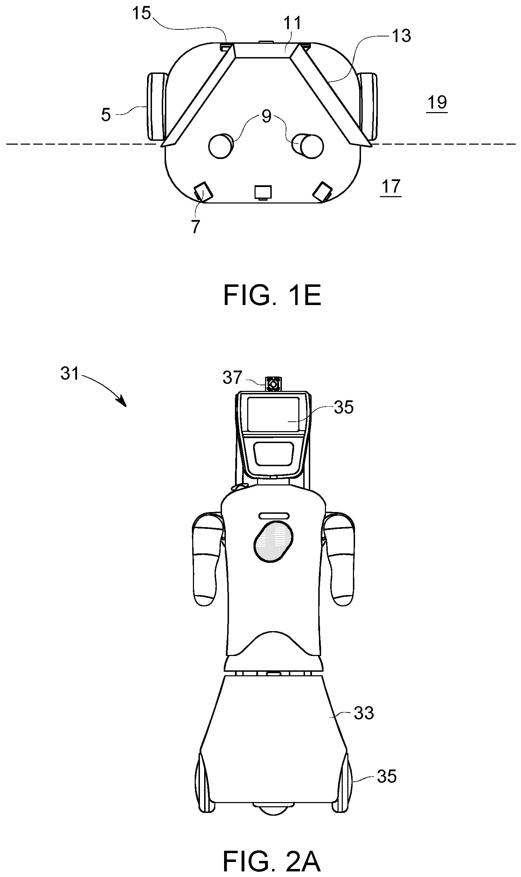

Figures 1a to 1e show an embodiment of the present invention which comprises a base 3 which has a three dependently movable wheels 5 which allow the device to move in a straight line forward and backwards and to turn clockwise or anticlockwise around a central axis of rotation. The base 3 also includes navigation sensors and associated software to enable autonomous, semi-autonomous or manually controlled (by a robot operator) navigation. In other embodiments, rollers or tracks, for example may be used in place of wheels.

The base 3 has a top surface upon which is mounted a pair of germicidal lamps 9. In this example of the present invention, the lamps 9 emit ultraviolet radiation in the range 200 -300 nanometres wavelength. The lamps shown in figures 1a to 1d extend vertically from the base 3. In other embodiments of the invention, more or fewer lamps may be used and they may be mounted horizontally or in another orientation. In addition, they may be mounted to the shield 13.

UV Shield 13 is mounted on the rear of the base 3 to extend around the base 3. As shown, the UV shield 13 is located adjacent to the UV lamps 9 and acts to shield one side of the robot 1 from UV light. The side of this shield adjacent to the UV lamp may have a reflective surface to amplify the light projected to the surrounding area. The area behind the robot, blocked by the shield is referred to the Light-Shielded Region (LSR) 19 and the area not blocked by the shield is referred to as the Light-Emission Region (LER) 17.

In other embodiments of the invention the size of the shield may be larger such that it encloses larger part of the base 5. In other embodiments a smaller shield which extends around a smaller part of the circumference of the base 5 may be used. The base itself may be rectangular, square or any other suitable shape.

Detectors 7 comprise a combination of sensors which may include cameras, passive infra-red, thermal vision sensors which are suitable for detecting the presence of a moving or stationary person or animal in the vicinity of the robot 1. Computer software, hardware and firmware are also provided. This control system regulates when the light is turned on/off, ensuring the light is turned off when people are close by. In at least one embodiment, where a person or animal is detected in the LSR region 19, the device may continue to emit UV radiation in the LER region 17.

A communications interface 15 is positioned on the rear of the shield. The communications interface sends and receives signals from controller and may comprise a computer screen, tablet computer or robotic head which is mounted to the outer part of the UV shield. The communication interface 15 may be fixed (i.e. rigidly attached to the UV shield) or connected via a movable articulated link (i.e. a 'neck-like' mechanism). In this embodiment, audio speakers are provided and may be housed at any location on the robot and would form part of the communication interface. The shield can also comprise an emergency stop button, one or more sensors and/or a gablet joystick holder (not shown). A communication beacon and/or spherical camera can be mounted on top of the unit (not shown).

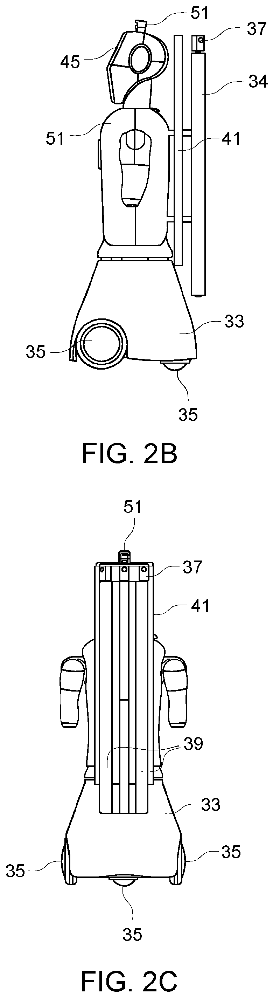

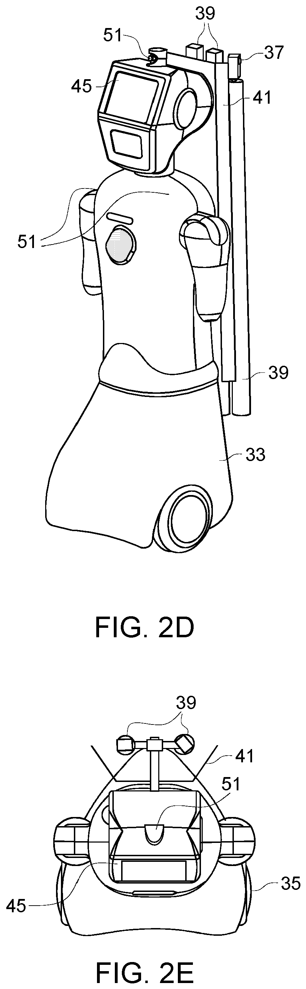

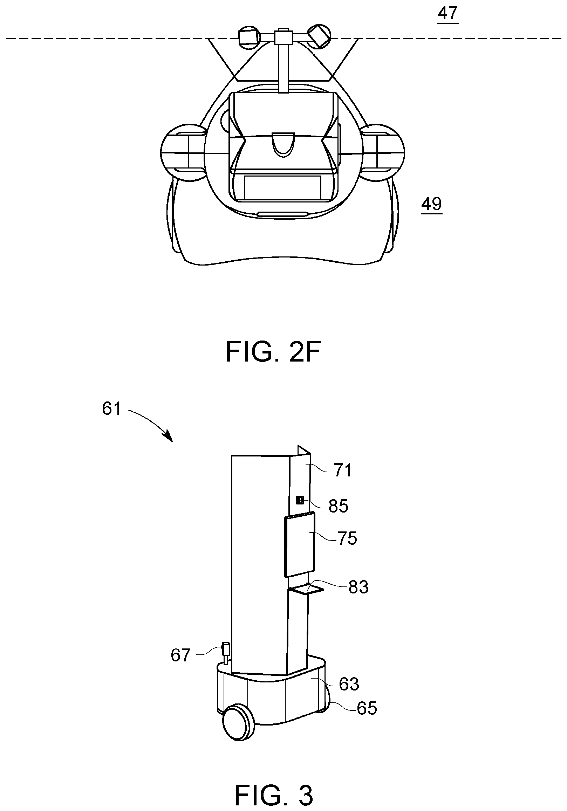

Figures 2a to 2f show a second embodiment of the present invention which comprises a robot 31 which comprises a base 33, wheels 35, LER side 47 detectors 37, UV lamps 39 a shield 41, a communications interface 45, which in this example is a screen mounted on an upper part of the base which resembles a head upon a torso. LSR side 49 detectors 51 are also shown.

Figure 3 is a front perspective view of a third embodiment of the present invention which comprises a robot 61 which comprises a base 63, wheels 65, LER side 77 detectors 67, UV lamps 69 a shield 71, a communications interface 75, which in this example is a screen mounted on the rear of the shield 71. A storage facility 83 is provided to enable the transporting of objects.

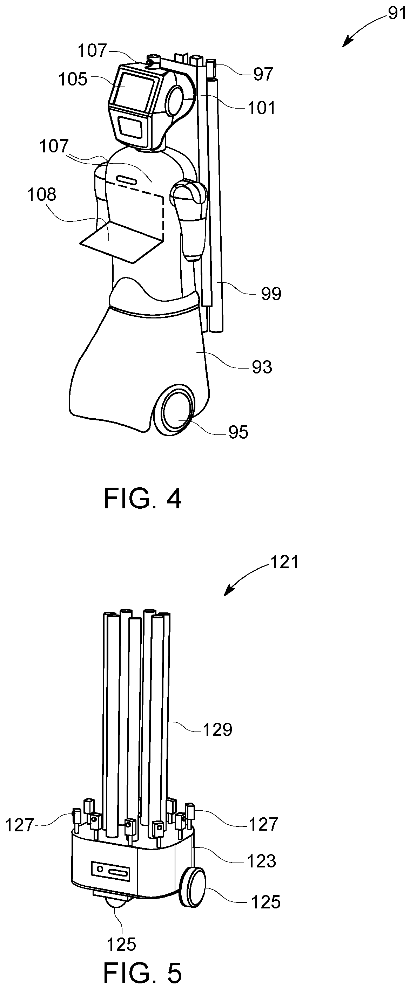

Figure 4 is a front perspective view of a fourth embodiment of the present invention which comprises a robot 91 which comprises a base 93, wheels 95, LER side detectors 97, UV lamps 99 a shield 101, a communications interface 108, which in this example is a screen mounted on an upper part of the base which resembles a head upon a torso. LSR side detectors 107 are also shown as well as a storage facility 108 to enable the transporting of objects.

Figure 5 is a front perspective view of a fifth embodiment of the present invention which comprises a robot 121 which comprises a base 123, wheels 125, detectors 127 and UV lamps 129.

In use, the apparatus of the present invention is embodied as an autonomous, semi-autonomous or remote-control robot. The robot may be used in performing disinfecting cleaning procedures in hospitals, nursing homes, and other settings where harmful microorganisms may be present. UV cleaning has distinct advantages over traditional chemical-based cleaning methods which are ineffective, and involve procedures that are labour intensive, environmentally harmful and hazardous for cleaning staff.

In one embodiment, the robot's sensors and control software allow it to determine the presence of obstacles within an environment and to navigate around them autonomously. The system is also adaptable for use in scenarios where the robot has been programmed with details of the layout of the building which is to be disinfected and can operate semi-autonomously. The robot may also be operated via a remote control in an environment and requires input from a human operator.

In the case where the robot has a map of its environment, it will use its software to compute an optimal trajectory through the space where it will stop for periods of time, turning the light on as appropriate to disinfect the space. The robot will not turn the light on if it detects a person or animal in the light emission region (LER). The robot may, from time to time, send a signal to a robot operator to let them know a room has been cleaned, report an error, request that a door is opened, etc. In the event that humans are detected during a cleaning procedure, the communication interface provides a focal point for communicating with them, this may be a request to vacate the space temporarily, or to warn if they approach that the robot is currently engaged in a cleaning task and to keep their distance.

In the case where the robot does not have a map of its environment, it will be closely supervised by a robot operator, who may manually control the robot using a joystick or some other controller embodiment. The operator may be physically located adjacent to the robot, or may be controlling the robot remotely, using the robots camera sensors and an internet connection to gain situational awareness of the robots surroundings.

In the event that the operator is physically present, they would stand in the LSR while navigating the robot, which would ensure that when the light is turned on, they would not be exposed to the IR light. The person/animal detection control system would be used as before to ensure that the light is only turned on when people are not in the LER.

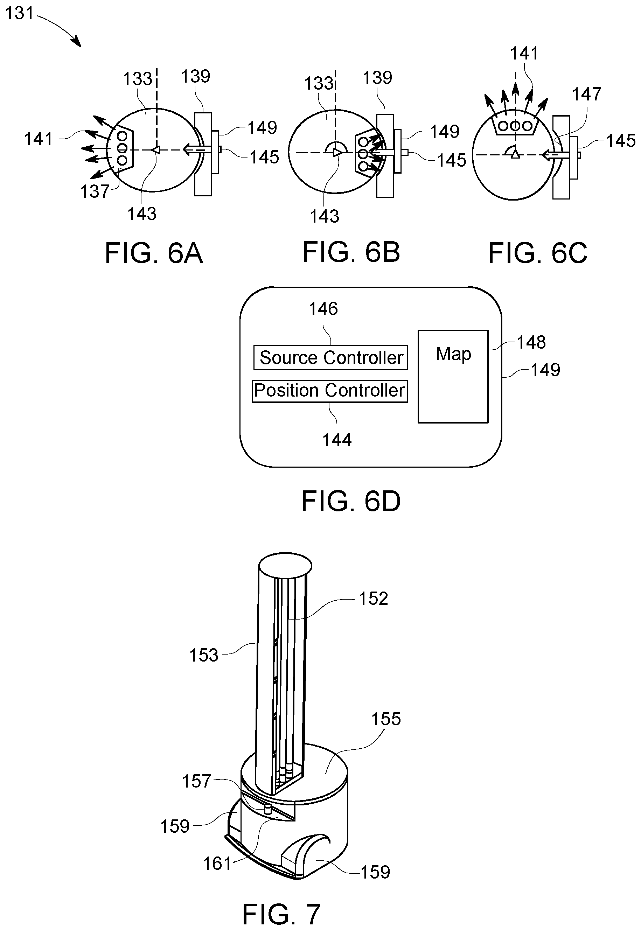

Figures 6a to 6d are schematic plan views of a first embodiment of an apparatus in accordance with the present invention shown in three distinct configurations and a user interface.

The embodiment 131 comprises a rotatable casing 133, a light source 135, which is partially surrounded by a blocking surface 137 which is preferably a surface reflective to UVC light. An enclosure column 139 further allows the light source to be surrounded. Emitted light 141 is shown along with the general direction of light emission 143 and the normal forward facing direction of the apparatus 145. A further detail is that the enclosure 149 has a curved surface 147 which is shaped to accommodate the substantially cylindrical rotatable casing 3. The user interface 149 is mounted on the rear of the device to allow a human tele-operator standing behind the robot to gain situational awareness on the surfaces being irradiated by the robot. It comprises a screen upon which a map 148 is displayed along side a source controller 146 and a position controller. The map 148 shows the user the position of the device relative to objects in the room.

In other embodiments of the present invention, the user interface may be implemented as a software application on a tablet computer or other suitable device to allow a human tele-operator to perform remote UV teleoperation procedure. From a usability and operations perspective, the communication interface plays an important role. The communication interface here refers primarily to the rear side of the apparatus. This provides a focal point for interaction with users that is shielded from UV light (i.e. even if the light rotated rearwardly, the UV irradiation is blocked from the person interacting with the robot).

In figure 6a, light 141 is emitted from the light source 135 in the forward-facing direction 143. In figure 6b, the blocking surface 137 has been rotated to face the enclosure column 139 and the light 141 is emitted from the light source 145 in a direction towards the enclosure column 139 such that the light is contained within the apparatus. In this configuration, the light source 135 is protected from damage by being enclosed in between the blocking barrier 137 and enclosure column 139. In figure 6c, light 141 is emitted from the light source 135 in a direction perpendicular to the forward-facing direction 145 of the device 131.

The device shown in figures 6a to 6d allows for selective irradiation of the area to be treated by blocking the emission of light in all but the desired direction using the blocking barrier 137 which is opaque and preferably reflective to UVC light. The blocking barrier can be dynamically reoriented by the robot to direct light in any arbitrary direction, independent of the orientation of the robot base or the direction in which the robot is travelling.

The enclosure column 139 or enclosure system, works with the blocking barrier 137 to completely or partially encapsulate the light source from the surrounding environment.

In the enclosed configuration (Figure 6 b), the light source is encapsulated from the environment using the enclosure column and blocking barrier. This protects the light source from damage and blocks light being irradiated from the light source.

Figure 7 is a perspective view of a second embodiment of the present invention. The device 151 comprises a UV source 152 which comprises three ultraviolet lamps positioned inside a blocking barrier 153 on top of a rotate table base 155. The base 155 has a set of wheels 159 which allow the device to move across a surface, and a sensor 157 which measures the position and distance of the device with reference to objects in its path. The sensor is located in a recess 161 on the front of the base 155. The blocking barrier 153 acts to block the emission of UVC light in undesired directions when the UVC source 152 is active and can be dynamically orientated by the robot during operation. The surface of the blocking barrier closest to the UVC source is preferably reflective to UVC

In at least one embodiment of the present invention the direction of the light from the light source 153 will be controlled so as to direct light towards the object which is nearest to the device 151 so as to increase the intensity of the germicidal ultraviolet radiation on an area of a surface in order to improve its effectiveness of the device 151.

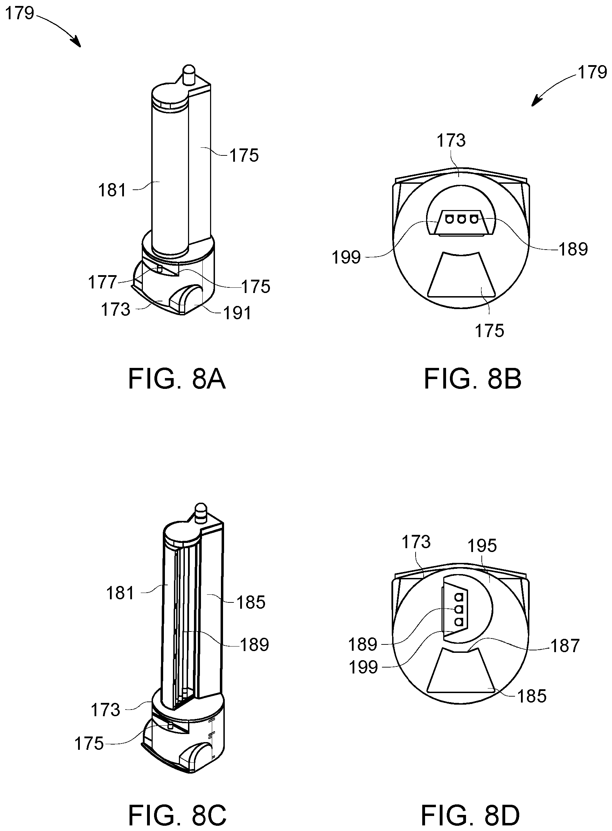

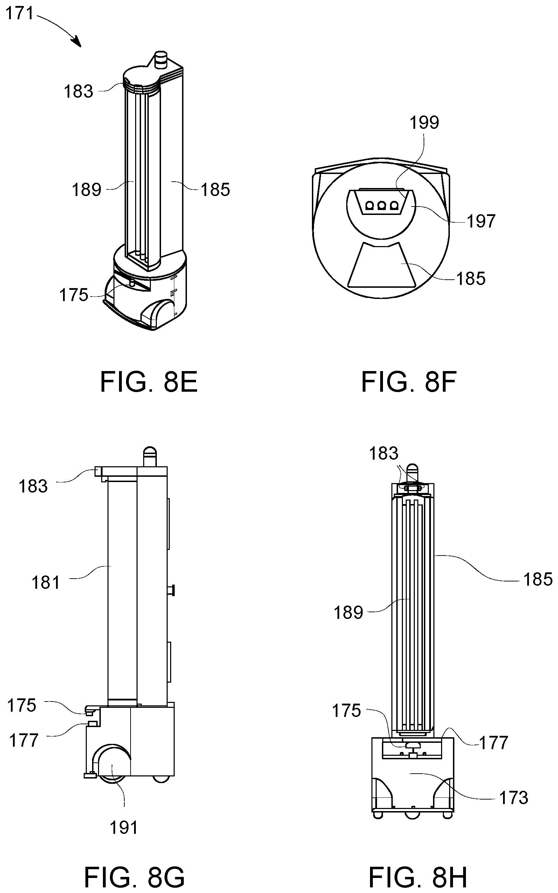

Figures 8a, 8c and 8e are perspective views of a third embodiment of the present invention, figures 8b, 8d and 8f are respective plan views and figures 8g and 8h are side and front views of the same embodiment. These figures show the device 171 which comprises a base 173 with a sensor 175 positioned in the front centre of the base within a recess 177. Figure 3a shows the movable cover 181 in a closed position such that the UV light source 189 is concealed within the cover 181. The enclosure column 185 is positioned to the rear of the cover 181 and acts to prevent UV radiation from being emitted from the rear of the device 171.

Figures 8 a to h also show wheels 191 which are positioned to the front of the device 171 a third wheel to the rear of the device is shown in figures g and h below the position of the blocking barrier 185. Figures 8a and 8b show the device in a closed position and which the casing 181 extends across the front of the device above sensor 175. Figure 8b which is a plan view of the device of figure 8a shows the position of the UV source 189 as it is pointed towards the enclosure column 185 with curved surface 187. In this configuration the light is contained within the device 171 and the lamps which form the UV light source are protected from mechanical wear and damage which could be caused during transportation of the device or the like.

Figures 8c and 8d show the embodiment of the present invention with the UV source 189 visible and pointing to the left of the forward-facing direction of the device 171. This has been achieved by a rotation of the unit which comprises the UV light source 189, the cover 181 and blocking surface 199 through approximately 90 degrees from the position as shown in figures 8a and 8b.

Figures 8e and 8f show the embodiment of the present invention with the UV source 189 visible and pointing in the forward-facing direction of the device 171. This has been achieved by a rotation of the unit which comprises the UV light source 189 the cover 181 and blocking barrier 199 through approximately 90 degrees from the position as shown in figures 8c and 8d. Figure 8g shows the configuration of the device from figures 8e and 8f as a side view and figure h shows the configuration of the device from figures 3e and f as a front view.

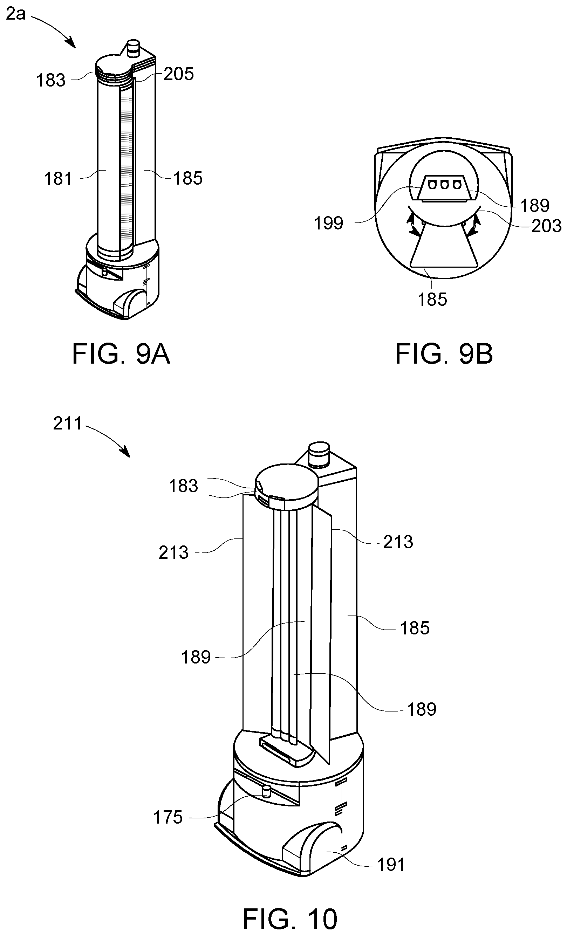

Figure 9a is a perspective views and 9b is a plan view of a fourth embodiment of the present invention. Figures 9a and 9b show a device 201 substantially similar to the device shown in figures 8a to g. In this example of the present invention, shutters 203 are fitted to the sides of the enclosure column 185. The shutters 83 extend down the length of the enclosure column and are fitted to the enclosure column by means of a hinge 205 which allows the shutters 203 be moved towards and away from the movable cover 181 so that the UV source is completely enclosed by means of the shutters.

Figure 10 is a front perspective view of a fifth embodiment of the present invention. In this example of the present invention an adjustable blocking barrier 213 is provided. This functions to allow the dynamic adjustment of the field of view of the ultraviolet source 189 such that the field of view maybe increased or decreased in size. This is of particular use when the object to be sterilised is positioned at a certain location with respect to the device 211 therefore the adjustable blocking barrier can narrow the field such that all of the UV radiation is concentrated on that specific object.

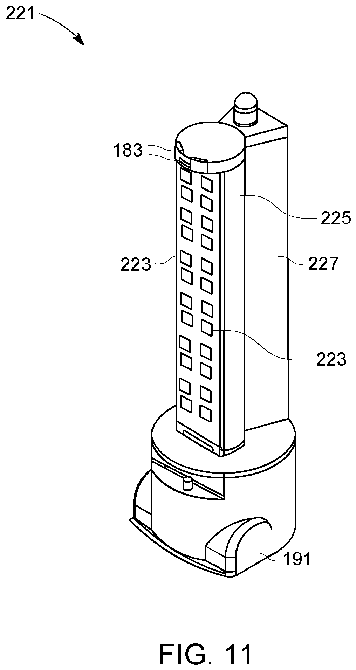

Figure 11 is a front perspective view of a sixth embodiment of the present invention. In this example of the present invention the device 221 has a UV source which comprises a plurality of light emitting diodes 223 which are mounted upon a flat surface 224 of movable LED support 225. Each of the LEDs comprise a single highly directional source of ultraviolet radiation which may be switched on individually. The moveable LED support 225 may be rotated 180° so that the light source is facing the blocking barrier 227.

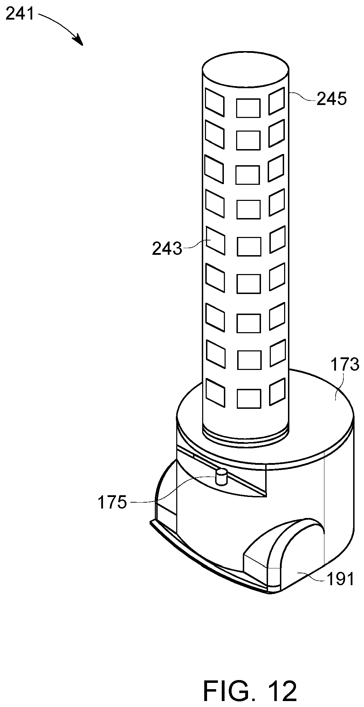

Figure 12 is a front perspective view of a seventh embodiment of the present invention. In this example of the present invention the device 241 has a UV source which comprises a plurality of light emitting diodes 243 which are mounted upon cylindrical support column 245. The LEDs are positioned around the column so that they face in multiple directions. Each of the LEDs comprise a single highly directional source of ultraviolet radiation which may be switched on individually. The moveable LED support 225 may be rotated 180° so that the light source is facing the blocking barrier 227.

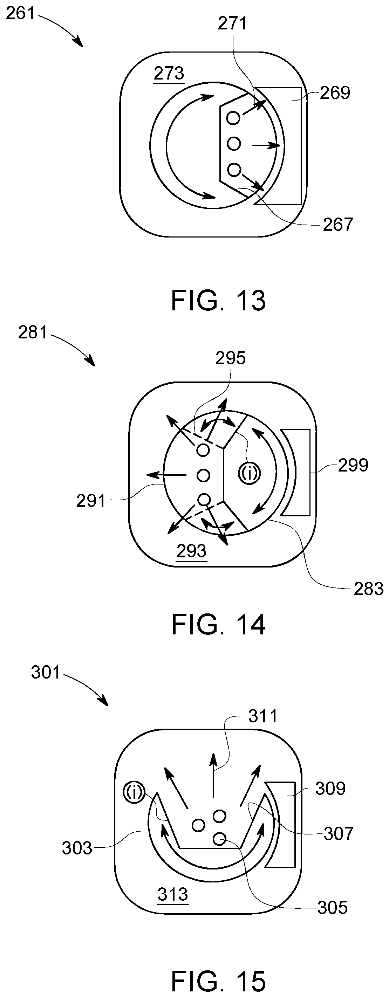

Figure 13 is a plan view of an eighth embodiment of the present invention.

Figure 13 shows the device 261 which has a similar general shape and configuration to that of figures 6a to g, with a rotatable casing 263, a light source 265, a light blocking barrier 267, an enclosure column 269 all mounted on a base 273. Arrows 271 show the direction in which light is emitted from the device 261. In this example, the enclosure column 149 is sized such that, when the light source 265 faces the blocking barrier the light source is completely encapsulated to prevent emission of UVC light.

Figure 14 is a plan view of a ninth embodiment of the present invention. Figure 14 shows the device 281 which has a similar general shape and configuration to that of figures 6a to g, with a rotatable casing 283, a light source 285 and a blocking barrier 291 all mounted on a base 293. Arrows 291 show the direction in which light is emitted from the device 281. In this embodiment, pivotable side members 295 which extend along the length of the rotatable casing adjust the angle over which light is emitted.

Figure 15 is a plan view of a tenth embodiment of the present invention. Figure 15 shows the device 301 which has a similar general shape and configuration to that of figures 6a to g, with a rotatable casing 303, a light source 305, a blocking barrier 307, an enclosure column 229 all mounted on a base 313. Arrows 311 show the direction in which light is emitted from the device 301. In this example of the invention, the light source 305 remains fixed to the base while the blocking surface/reflector 307 rotates around it to direct light 311.

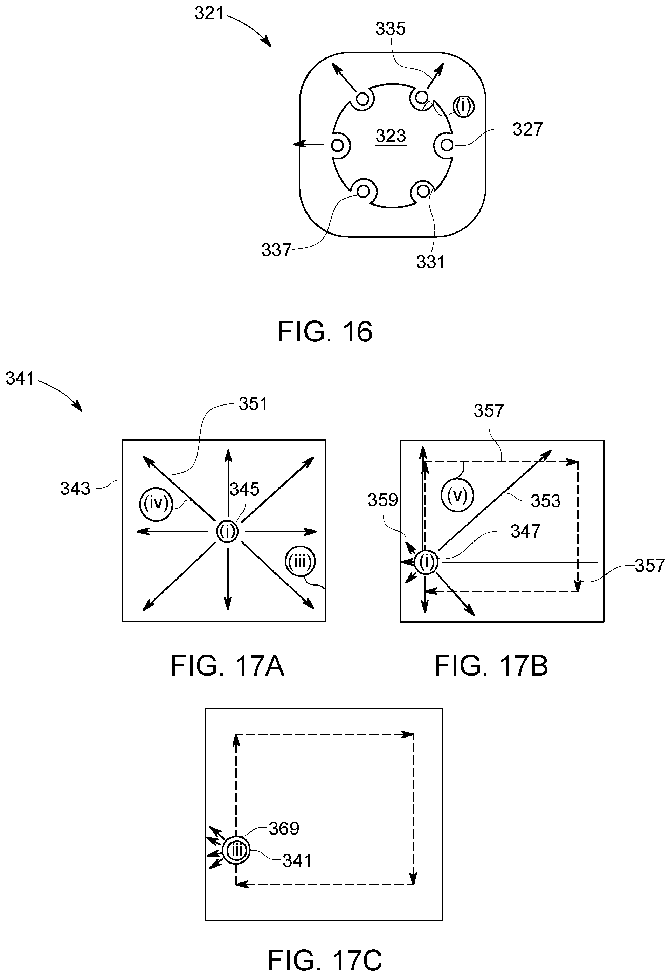

Figure 16 is a plan view of an eleventh embodiment of the present invention. Figure 16 shows the device 321 which has a similar general shape and configuration to that of figure 12 in that it has a cylindrical column 323 to which sources of germicidal UV light are attached. In this example, UV fluorescent lamps 327 are contained in recesses 331 arranged around the circumference of column 323. The lights positioned on the robot are selectively illuminated, in this example lights 335 are on and lights 2337 are off, this allows the light to be directed in a preferred direction.

Figures 17a and 17 b show typical operation of a traditional device for the disinfection of a room with UVC using a robot. Figure 17 c shows how this may be improved using a strategy possible with an apparatus in accordance with the present invention

The figures show a device 341 within a room 23. The device 341 is shown at three locations 345, 377 and 9 in figures 17a, b and c respectively.

Figure 17a shows the device 341 in central position 345 with UV radiation emitted 351 omnidirectionally around the room from that position. Figure 17b shows the device 341 in position 347 and on a pathway 357 around the room. Arrows 359 show a short path from the device to a surface adjacent to the device 261 and a long path to the surface is remote from the device 353. It will be noted that the intensity of radiation against the surface which is close to the device will be higher. In figure 17c, the device 341 is shown in position 369 and follows path 279. In this example all of the radiation is directed to the surface in close proximity to the device therefore the intensity of the radiation against that surface is higher. The arrangement shown in figures 17a to 17c may form the basis of the map provided on an example of a graphical user interface in one or more example of the present invention.

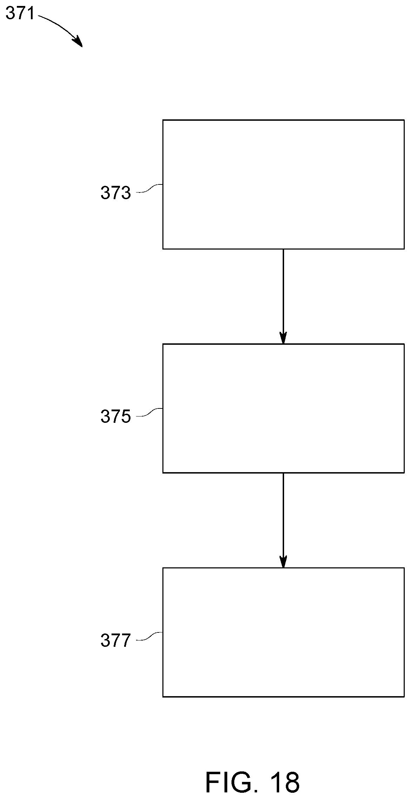

Figure 18 is a flow diagram 371 of an example of the method for controlling the apparatus in accordance with the present invention implemented on computing means. The sensors firstly detect the position and distance of one or more objects with respect to the apparatus as well as the location of people in the vicinity 373, a calculation 375 is made to determine which of said objects are closest to the device, in response to this calculation, the output of the germicidal light source is controlled 377 such that germicidal light is directed towards said closest object and away from people.

The present invention provides for disinfection using germicidal radiation such as UVC using a controlled, semi-autonomous or autonomous device such as a robot.

In examples, the device can irradiate a room selectively using actuated germicidal radiation in the form of light columns and blocking barriers without a person being present.

In some embodiments, radiation source may rotate independently of the mobile base to allow parts of the room to be irradiated independently of direction of forward motion.

A control system is provided for dynamically avoiding irradiating people using a combination of a rotatable radiation source which prevents radiation from exiting the device and adjusting UV lamp output (i.e. turning it off) in response to sensor data.

Shielding column allow a radiation source to be safely turned without risk of exposure until they reach their steady state value. The shielding column may also allow UV lamps to be safely turned on whilst they shield from people, without having to turn off the radiation source.

The present invention may also be provided with a dedicated interface on the device which allows a human tele-operator standing behind the device to gain situational awareness on the surfaces being irradiated by the robot. The interface on the device may also have an interface for a human tele-operator to perform remote UV teleoperation procedures. These features may be implemented using a tablet computer.

The primary envisioned application for the technology is in performing disinfecting cleaning procedures in hospitals, nursing homes, and other settings where harmful microorganisms may be present. UV cleaning has distinct advantages over traditional chemical-based cleaning methods which are ineffective, and involve procedures that are labour intensive, environmentally harmful and hazardous for cleaning staff. In particular, the embodiments documented here make it feasible for the device of the present invention to operate and irradiate UVC while in the same vicinity as people. This opens new commercial possibilities such as using the device to disinfect a room while it is being cleaned manually using traditional means or in public spaces where the device can safely operate while not directing UVC towards people. The enhanced efficiency of the device in terms of both the time required for disinfection and energy consumption as a result of selectively directing UVC towards surfaces close to the device has several commercial advantages. Additionally the ability to allow the light source to be turned on and reach nominal output prior to the disinfection treatment shortens the cleaning cycle, facilitating cleaning of environments when they are only available for short periods

In a preferred embodiment, the device is implemented as an autonomous robot which may be used to perform disinfecting cleaning procedures in hospitals, nursing homes, and other settings where harmful microorganisms may be present. UV cleaning has distinct advantages over traditional chemical-based cleaning methods which are ineffective, and involve procedures that are labour intensive, environmentally harmful and hazardous for cleaning staff.

Some of the embodiments of the invention described with reference to the drawings comprise a method implemented on computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, or magnetic recording medium, e.g. a memory stick or hard disk. The carrier may be an electrical or optical signal which may be transmitted via an electrical or an optical cable or by radio or other means.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A disinfection apparatus comprising:
a germicidal radiation source;
a moveable support to which the germicidal light source is attached which is moveable in the environment; a controller for controlling emission of germicidal light radiated omnidirectionally from the germicidal light source wherein,
the controller comprises a germicidal light blocker and a control system so as to selectively block light emitted from the light source in selected directions and direct it in other directions;
the germicidal radiation blocker comprises:
• a first barrier which is fixed in position with respect, and moves with the germicidal radiation source and has a reflecting surface which reflects germicidal radiation in a direction to and out from a second side of the germicidal radiation source, the radiation source and first barrier being rotatable relative to;
• a second barrier which is fixed relative to the movable support wherein the first barrier and the second barrier work together to completely or partially encapsulate the light source with respect to its surrounding environment.

2. The disinfection apparatus as claimed in claim 1 wherein, a communication interface comprising a user interface mounted on the second barrier at the rear of the device in a radiation shielded region (RSR) such that an operator can stand in the RSR to operate the user interface without being exposed to radiation.

3. The apparatus as claimed any previous claims wherein the controller further comprises a detector which uses a plurality of sensors to detect the presence of an object or people in the vicinity of the apparatus wherein the controller controls the direction of germicidal light from the light source in response to the detection of one or more said objects or people.

4. The apparatus as claimed in any preceding claim wherein, the controller comprises one or more proximity detectors which measures the position of and distance between an object and the apparatus, said distance and position measurements are used to control the direction in which germicidal light is emitted from the light source such that the germicidal light is concentrated on the object.

5. The disinfection apparatus as claimed in any preceding claim wherein the environment of the device is mapped such that the device uses software to compute an optimal trajectory through the space where it will stop for periods of time, irradiating surfaces from different perspectives as appropriate to disinfect the space.

6. The disinfection apparatus as claimed in claim 2 wherein, the user interface comprises a screen upon which a map is displayed along side a source controller and a position controller, wherein the map shows the user the position of the device relative to objects in the room .

7. The disinfection apparatus as claimed any previous claims wherein, the user interface includes a speaker.

8. The apparatus as claimed in any preceding claim wherein, the second barrier is rotatable or fixed with respect to the moveable support

9. The apparatus as claimed in any preceding claim wherein, the germicidal light source comprises a plurality of LEDs arranged on a support surface, wherein the controller comprises a switch which controls the operation of the LEDs.

10. The apparatus as claimed in in any preceding claim wherein the germicidal light source comprises a plurality of fixed light sources positioned on a support column and facing in multiple directions.

11. The apparatus as claimed in any preceding claim which comprises a robotic system.

12. The apparatus as claimed in any preceding claim wherein, the germicidal light source is UV radiation in the range of 200-300 nm.

13. The apparatus as claimed in any preceding claim wherein, the moveable support comprises a moveable base.

14. The apparatus as claimed in any preceding claim wherein, the apparatus further comprises navigation sensors which detect the presence of objects in the pathway of the apparatus.

15. A method for controlling the apparatus as claimed in claims 1 to 14, the method comprising:
detecting the position and distance of one or more objects with respect to the apparatus;
detecting if a person is in the vicinity of the apparatus determining which of said objects are closest to the apparatus;
controlling the output of a germicidal light source such that germicidal light is (a) directed towards said closest object and (b) when a person is detected in the vicinity of the apparatus, directed away from the person.

16. A computer program with program instructions or implementing the method of claim 16.
